Europäisches Patentamt

European Patent Office

Office européen des brevets

(19)

(11) Publication number: **0 282 185**
**A1**

## (12) EUROPEAN PATENT APPLICATION

(21) Application number: 88301349.2

(22) Date of filing: 18.02.88

(51) Int. Cl.⁴: **C07K 13/00** , C12N 15/00 ,
C12P 21/02 , A61K 37/02

(30) Priority: 18.02.87 US 16079

(43) Date of publication of application:
14.09.88 Bulletin 88/37

(84) Designated Contracting States:
ES GR

(71) Applicant: **Schering Biotech Corporation**
**901 California Avenue**
**Palo Alto California 94304-1104(US)**

(72) Inventor: **Otsuk, Takeshi**
**301, 3-25-1 Hirao Chou-ku**
**Fukuoka 81D(JP)**

(74) Representative: **Ritter, Stephen David et al**
**Mathys & Squire 10 Fleet Street**
**London EC4Y 1AY(GB)**

(54) **Human interleukin-3 and muteins thereof.**

(57) Novel human interleukin-3 proteins are provided which contain a non-conservative amino acid substitution (Ser --> Pro at position 7) with respect to previously reported sequences for human IL-3. Methods for synthesizing conformationally and antigenically neutral muteins are also provided. The disclosed human IL-3 protein promotes the growth and development of a wide variety of hematopoietic lineages, and may be useful in treating conditions involving depressed blood cell regeneration and/or populations, such as myeloid hypoplasia, chronic infections, and therapies using transplanted bone marrows.
The sequence is as follows:

X(Pro) - X(Met) - X(Thr) - X(Gln) - X(Thr) - W(Thr) -
10
W(Pro) - W(Leu) - W(Lys) - X(Thr) - X(Ser) - X(Trp) -

X(Val) - X(Asn) - Cys - X(Ser) - W(Asn) - W(Met) -
20
W(Ile) - W(Asp) - W(Glu) - W(Ile) - W(Ile) - W(Thr) -
30
W(His) - W(Leu) - W(Lys) - W(Gln) - W(Pro) - W(Pro) -

X(Leu) - X(Pro) - X(Leu) - X(Leu) - X(Asp) - X(Phe) -
40
X(Asn) - X(Asn) - W(Leu) - W(Asn) - W(Gly) - W(Glu) -

W(Asp) - W(Gln) - W(Asp) - W(Ile) - W(Leu) - W(Met) -
50
W(Glu) - W(Asn) - W(Asn) - W(Leu) - W(Arg) - W(Arg) -
60
W(Pro) - W(Asn) - W(Leu) - W(Glu) - W(Ala) - W(Phe) -

W(Asn) - W(Arg) - W(Ala) - W(Val) - W(Lys) - W(Ser) -
70
W(Leu) - W(Gln) - W(Asn) - W(Ala) - W(Ser) - W(Ala) -

W(Ile) - W(Glu) - W(Ser) - W(Ile) - W(Leu) - X(Lys) -
80
X(Asn) - X(Leu) - X(Leu) - X(Pro) - Cys - X(Leu) -
90
X(Pro) - X(Leu) - X(Ala) - X(Thr) - X(Ala) - W(Ala) -

W(Pro) - W(Thr) - W(Arg) - W(His) - W(Pro) - W(Ile) -
100
W(His) - W(Ile) - W(Lys) - W(Asp) - W(Gly) - W(Asp) -

W(Trp) - W(Asn) - W(Glu) - W(Phe) - W(Arg) - W(Arg) -
110
W(Lys) - W(Leu) - X(Thr) - X(Phe) - X(Tyr) - X(Leu) -
120
X(Lys) - W(Thr) - W(Leu) - W(Glu) - W(Asn) - W(Ala) -

W(Gln) - W(Ala) - W(Gln) - W(Gln) - X(Thr) - X(Thr) -
130
X(Leu) - X(Ser) - X(Leu) - X(Ala) - X(Ile) - X(Phe)

EP 0 282 185 A1

X(Pro) - X(Met) - X(Thr) - X(Gln) - X(Thr) - W(Thr) -

W(Pro) - W(Leu) - W(Lys) - X(Thr) - X(Ser) - X(Trp) -

X(Val) - X(Asn) - Cys - X(Ser) - W(Asn) - W(Met) -

W(Ile) - W(Asp) - W(Glu) - W(Ile) - W(Ile) - W(Thr) -

W(His) - W(Leu) - W(Lys) - W(Gln) - W(Pro) - W(Pro) -

X(Leu) - X(Pro) - X(Leu) - X(Leu) - X(Asp) - X(Phe) -

X(Asn) - X(Asn) - W(Leu) - W(Asn) - W(Gly) - W(Glu) -

W(Asp) - W(Gln) - W(Asp) - W(Ile) - W(Leu) - W(Met) -

W(Glu) - W(Asn) - W(Asn) - W(Leu) - W(Arg) - W(Arg) -

W(Pro) - W(Asn) - W(Leu) - W(Glu) - W(Ala) - W(Phe) -

W(Asn) - W(Arg) - W(Ala) - W(Val) - W(Lys) - W(Ser) -

W(Leu) - W(Gln) - W(Asn) - W(Ala) - W(Ser) - W(Ala) -

W(Ile) - W(Glu) - W(Ser) - W(Ile) - W(Leu) - X(Lys) -

X(Asn) - X(Leu) - X(Leu) - X(Pro) - Cys - X(Leu) -

X(Pro) - X(Leu) - X(Ala) - X(Thr) - X(Ala) - W(Ala) -

W(Pro) - W(Thr) - W(Arg) - W(His) - W(Pro) - W(Ile) -

W(His) - W(Ile) - W(Lys) - W(Asp) - W(Gly) - W(Asp) -

W(Trp) - W(Asn) - W(Glu) - W(Phe) - W(Arg) - W(Arg) -

W(Lys) - W(Leu) - X(Thr) - X(Phe) - X(Tyr) - X(Leu) -

X(Lys) - W(Thr) - W(Leu) - W(Glu) - W(Asn) - W(Ala) -

W(Gln) - W(Ala) - W(Gln) - W(Gln) - X(Thr) - X(Thr) -

X(Leu) - X(Ser) - X(Leu) - X(Ala) - X(Ile) - X(Phe)

## HUMAN INTERLEUKIN-3 AND MUTEINS THEREOF

The invention generally relates to protein mediators of hematopoietic system development, and to nucleic acids coding therefor. More particularly, the invention relates to a variant of human interleukin-3, to muteins thereof, and to their encoding nucleic acids.

Circulating blood cells are constantly replaced by newly developed cells. Replacement blood cells are formed in a process termed hematopoiesis which involves the production of at least eight mature blood cell lineages: red blood cells (erythrocytes), macrophages (monocytes), eosinophilic granulocytes, megakaryocytes (platelets), neutrophilic granulocytes, basophilic granulocytes (mast cells), T lymphocytes, and B lymphocytes (Burgess and Nicola, Growth Factors and Stem Cells (Academic Press, New York, 1983)). Much of the control of blood cell formation is mediated by a group of interacting glycoproteins termed colony stimulating factors (CSFs). These glycoproteins are so named because of the in vivo and in vitro assays used to detect their presence. Techniques for the clonal culture of hematopoietic cells in semisolid culture medium have been especially important in the development of in vitro assays. In such cultures, individual progenitor cells (i.e., cells developmentally committed to a particular lineage, but still capable of proliferation) are able to proliferate to form a colony of maturing progeny in a manner which is believed to be essentially identical to the comparable process in vivo. The role of CSFs in hematopoiesis is the subject of many recent reviews, e.g. Metcalf, The Hemopoietic Colony Stimulating Factors (Elsevier, New York, 1984); Metcalf, Science, Vol. 229, pgs. 16-22 (1985); Nicola et al., Immunology Today, Vol. 5, pgs. 76-80 (1984); and Whetton et al., TIBS, Vol. 11, pgs. 207-211 (1986).

The detection, isolation and purification of these factors is extremely difficult, for it is frequently complicated by the nature of the supernatants they are typically located in, the divergencies and cross-overs of activities of the various components in the mixtures, the sensitivity (or lack thereof) of the assays utilized to ascertain the factors' properties, the frequent similarity in the range of molecular weights and other characteristics of the factors, and the very low concentration of the factors in their natural settings.

As more CSFs become available, primarily through molecular cloning, interest has heightened in finding clinical applications for them. Because of physiological similarities to hormones (e.g., soluble factors, growth mediators, action via cell receptors), potential uses of CSFs have been analogized to the current uses of hormones, e.g. Dexter, Nature, Vol. 321, pg. 198 (1986). Their use has been suggested for several clinical situations where the stimulation of blood cell generation would be desirable, such as for rehabilitative therapy after chemotherapy or radiation therapy of tumors, treatment of myeloid hypoplasias, treatment of neutrophil deficiency, treatment to enhance hematopoietic regeneration following bone marrow transplantation, and treatment to increase host resistance to established infections, e.g. Dexter (cited above), and Metcalf, Science (cited above).

Over the past several years a murine CSF activity, termed multi-CSF or interleukin-3 (IL-3), has been identified, purified, and cloned; see Yokota et al; Proc. Natl. Acad. Sci., Vol. 81, pgs. 1070-1074 (1984); Fung et al., Nature, Vol. 307, pgs. 233-237 (1984); Hapel et al., Blood, Vol. 65, pgs. 1453-1459 (1985); Ihle et al., J. Immunol., Vol. 129, pgs. 2431-2436 (1982); Ihle et al., J. Immunol., Vol. 131, pgs. 282-287 (1983); and Iscove et al., pgs. 397-425 in Cellular and Molecular Biology of Lymphokines (Academic Press, New York, 1985). Murine IL-3 exhibits a broad spectrum of CSF activities, being capable of stimulating proliferation and development of the progenitor cells of all the recognized myeloid lineages, e.g. Dexter, Nature, Vol. 309, pgs. 746-747 (1984); Ihle et al., Lymphokines, Vol. 9, pgs. 153-200 (1984). Because of the broad stimulatory effects of the factor, there has been a great deal of interest in isolating the analogous factors in other mammals, particularly man.

Recently, rat, gibbon, and human homologues of mouse IL-3 have been reported by Cohen et al., Nucleic Acids Research, Vol. 14, pgs. 3641-3658 (1986); and Yang et al., Cell, Vol. 47, pgs. 3-10 (1986). The amino acid sequences of the reported molecules have a relatively low degree of homology to mouse IL-3: 54 percent for rat, 29 percent for gibbon, and 29 percent for human. Moreover it is still not certain whether the human homologue possesses the same range of biological activities as its mouse counterpart.

In view of the foregoing, the availability of variants of the human homologue of mouse IL-3 with confirmed biological activities would be highly advantageous for possible clinical uses, particularly in situations where the stimulation of blood cell generation could compensate disease-induced myeloid hypoplasia, reverse the deleterious side effects of cancer therapies, enhance the efficacy of bone marrow transplants, or the like.

The present invention is directed to a human interleukin-3 (IL-3) and genetically engineered muteins thereof. It includes polypeptides which comprise a sequence of amino acids selected from the set defined below by Formula I, and which possess a colony stimulating factor (CSF) activity as defined by standard assays. The invention also includes nucleic acids capable of encoding the polypeptides defined by Formula I, methods for making such polypeptides by use of the nucleic acids of the invention, and methods of using the polypeptides of the invention either alone or with other CSFs to treat disorders involving myeloid hypoplasia or to enhance hematopoietic system regeneration following bone marrow transplantation or following certain cancer therapies.

A preferred embodiment of the invention is the set of glycosylated or unglycosylated polypeptides which comprise a sequence of amino acids selected from the set of sequences defined by the following formula:

X(Pro) — X(Met) — X(Thr) — X(Gln) — X(Thr) — W(Thr) —
10

W(Pro) — W(Leu) — W(Lys) — X(Thr) — X(Ser) — X(Trp) —

X(Val) — X(Asn) — Cys — X(Ser) — W(Asn) — W(Met) —
20

W(Ile) — W(Asp) — W(Glu) — W(Ile) — W(Ile) — W(Thr) —
30

W(His) — W(Leu) — W(Lys) — W(Gln) — W(Pro) — W(Pro) —

X(Leu) — X(Pro) — X(Leu) — X(Leu) — X(Asp) — X(Phe) —
40

X(Asn) — X(Asn) — W(Leu) — W(Asn) — W(Gly) — W(Glu) —

W(Asp) — W(Gln) — W(Asp) — W(Ile) — W(Leu) — W(Met) —
50

W(Glu) — W(Asn) — W(Asn) — W(Leu) — W(Arg) — W(Arg) —
60

W(Pro) — W(Asn) — W(Leu) — W(Glu) — W(Ala) — W(Phe) —

W(Asn) — W(Arg) — W(Ala) — W(Val) — W(Lys) — W(Ser) —
70

W(Leu) — W(Gln) — W(Asn) — W(Ala) — W(Ser) — W(Ala) —

W(Ile) — W(Glu) — W(Ser) — W(Ile) — W(Leu) — X(Lys) —
80

X(Asn) — X(Leu) — X(Leu) — X(Pro) — Cys — X(Leu) —
90

X(Pro) — X(Leu) — X(Ala) — X(Thr) — X(Ala) — W(Ala) —

W(Pro) — W(Thr) — W(Arg) — W(His) — W(Pro) — W(Ile) —
100

W(His) — W(Ile) — W(Lys) — W(Asp) — W(Gly) — W(Asp) —

W(Trp) — W(Asn) — W(Glu) — W(Phe) — W(Arg) — W(Arg) —
110

W(Lys) — W(Leu) — X(Thr) — X(Phe) — X(Tyr) — X(Leu) —
120

X(Lys) — W(Thr) — W(Leu) — W(Glu) — W(Asn) — W(Ala) —

W(Gln) — W(Ala) — W(Gln) — W(Gln) — X(Thr) — X(Thr) —
130

X(Leu) — X(Ser) — X(Leu) — X(Ala) — X(Ile) — X(Phe)


Formula I

wherein the terms W(Xaa) and X(xaa) represent groups of synonymous amino acids to the amino acid Xaa located in a hydrophilic or a hydrophobic region of the protein, respectively, as determined by Hopp-Woods analysis. Synonymous amino acids within a group have sufficiently similar physicochemical properties for substitution between members of the group to preserve the biological function of the molecule: Grantham, Science, vol. 185, pgs. 862-864 (1974); and Dayhoff et al., "A Model of Evolutionary Change in Proteins", Atlas of Protein Sequence and Structure 1972, Vol. 5, pgs. 89-99. It is clear that insertions and deletions of amino acids may also be made in the above-defined sequence without altering biological function, particularly if the insertions or deletions only involve a few amino acids, e.g. under ten, and do not remove or displace amino acids which are critical to a functional conformation, e.g. cysteine residues: Anfinsen, "Principles That Govern The Folding of Protein Chains", Science, Vol. 181, pgs. 223-230 (1973). Proteins and muteins produced by such deletions and/or insertions come within the purview of the present invention. Whenever amino acid residues of the protein of Formula I are referred to herein by number, such number or numbers are in reference to the N-terminus of the protein.

It should be noted that the human IL-3 polypeptide represented by the actual amino acids Xaa in formula I (and given later herein as formula II) contains a non-conservative amino acid substitution of proline for serine at position 7 with respect to sequences for human IL-3 previously reported by Yang et al. in Cell, Vol. 47, pgs. 3-10 (1986).

Preferably, the synonymous amino acid groups are those defined in Tables I and II. More preferably, the synonymous amino acid groups are those listed before the second slash in each line in Tables I and II; and most preferably the synonymous amino acid groups are those listed before the first slash in each line in Tables I and II (each most preferred W(Xaa) group consisting of just Xaa itself).

Table I.  Preferred, More Preferred and Most Preferred
Groups of Synonymous Amino Acids X(Xaa)

| Xaa | X(Xaa) |
|-----|--------|
| Ser | Ser,//Thr, Gly, Asn, Cys |
| Arg | Arg,/Lys, His,/Gln, Glu |
| Leu | Leu, Ile, Met,/Phe/Val, Tyr |
| Pro | Pro,/Ala,/Gly, Thr |
| Thr | Thr,//Pro, Ser, Ala, Gly, His, Gln |
| Ala | Ala,/Pro,/Gly, Thr |
| Val | Val,/Met, Ile,/Leu, Tyr, Phe |
| Gly | Gly,//Ala, Thr, Pro, Ser |
| Ile | Ile, Met, Leu,/Phe, Val,/Tyr |
| Phe | Phe,/Met, Tyr, Ile, Leu,/Val, Trp |
| Tyr | Tyr,/Phe,/Trp, Met, Ile, Val, Leu |
| His | His,/Gln, Arg,/Glu, Lys, Thr |
| Gln | Gln,/Glu, His,/Thr, Arg, Lys, Asn |
| Asn | Asn,/Asp,/Ser, Gln |
| Lys | Lys,/Arg,/Glu, Gln, His |
| Asp | Asp,/Asn,/Glu |
| Glu | Glu,/Gln,/His, Arg, Asp, Lys, Asn |
| Met | Met, Ile, Leu,/Phe, Val/ |
| Trp | Trp// |
| Cys | Cys// |

## Table II. Preferred, More Preferred and Most Preferred Groups of Synonymous Amino Acids W(Xaa)

| Xaa | W(Xaa) |
|-----|--------|
| Ser | Ser,//Cys |
| Arg | Arg,//His, Lys |
| Leu | Leu,/Ile, Met,/Phe |
| Pro | Pro,//Ala |
| Thr | Thr// |
| Ala | Ala,//Pro |
| Val | Val,//Met, Ile |
| Gly | Gly// |
| Ile | Ile,/Met, Leu,/Phe, Val |
| Phe | Phe,//Met, Tyr, Ile, Leu |
| Tyr | Tyr,//Phe |
| His | His,//Gln, Arg |
| Gln | Gln,//Glu, His |
| Asn | Asn,//Asp |
| Lys | Lys,//Arg |
| Asp | Asp,//Asn |
| Glu | Glu,//Gln |
| Met | Met,/Ile, Leu,/Phe, Val |
| Trp | Trp// |
| Cys | Cys// |

The invention includes the polypeptides of Formula I with amino acid substitutions (between an amino acid of the polypeptide defined by Formula II (the putative native sequence) and a synonymous amino acid) at a single position or at multiple positions. The term "N-fold substituted" is used to describe a subset of polypeptides defined by Formula I wherein synonymous amino acids have been substituted for 0-N amino acids of the native sequence. Thus, for example, the group of 1-fold substituted polypeptides of Formula I consists of 381 polypeptides for the preferred groups of synonymous amino acids, 245 for the more preferred groups of synonymous amino acids, and 38 for the most preferred groups of synonymous amino acids. These numbers are arrived at by adding two sums: (i) the sum of the number of amino acids of each kind in the hydrophobic regions of the native sequence times the size of the synonymous amino acid group (X group) for that amino acid, and (ii) the sum of the number of amino acids of each kind in the hydrophilic regions of the native sequence times the size of the synonymous amino acid group (W group) for that amino acid.

Preferably the group of human IL-3 polypeptides consists of the 10-fold substituted polypeptides of Formula I wherein the amino acids in the regions defined by residues 10-32 inclusive, 47-60 inclusive, 71-86 inclusive, and 95-113 inclusive are not more than 3-fold substituted. The amino acid sequences in these regions are more highly conserved relative to the rest of the molecule when compared to mouse, rat, and gibbon IL-3 sequences.

More preferably, the group of human IL-3 polypeptides consists of the 5-fold substituted polypeptides of Formula I wherein the amino acids in the regions defined by residues 10-32 inclusive, 47-60 inclusive, 71-86 inclusive, and 95-113 inclusive are not more than 1-fold substituted. Still more preferably, the group of human IL-3 polypeptides consists of the 2-fold substituted polypeptides of Formula I wherein the amino acids in the regions defined by residues 10-32 inclusive, 47-60 inclusive, 71-86 inclusive, and 95-113 inclusive undergo no substitutions. Most preferably, the polypeptide of the invention comprises the native amino acid sequence defined by the following formula:

```
Pro - Met - Thr - Gln - Thr - Thr - Pro - Leu -
      10
Lys - Thr - Ser - Trp - Val - Asn - Cys - Ser -
                  20
Asn - Met - Ile - Asp - Glu - Ile - Ile - Thr -
                              30
His - Leu - Lys - Gln - Pro - Pro - Leu - Pro -
                                          40
Leu - Leu - Asp - Phe - Asn - Asn - Leu - Asn -

Gly - Glu - Asp - Gln - Asp - Ile - Leu - Met -
      50
Glu - Asn - Asn - Leu - Arg - Arg - Pro - Asn -
                  60
Leu - Glu - Ala - Phe - Asn - Arg - Ala - Val -
                              70
Lys - Ser - Leu - Gln - Asn - Ala - Ser - Ala -
                                          80
Ile - Glu - Ser - Ile - Leu - Lys - Asn - Leu -

Leu - Pro - Cys - Leu - Pro - Leu - Ala - Thr -
      90
Ala - Ala - Pro - Thr - Arg - His - Pro - Ile -
                  100
His - Ile - Lys - Asp - Gly - Asp - Trp - Asn -
                              110
Glu - Phe - Arg - Arg - Lys - Leu - Thr - Phe -
                                          120
Tyr - Leu - Lys - Thr - Leu - Glu - Asn - Ala -

Gln - Ala - Gln - Gln - Thr - Thr - Leu - Ser -
      130
Leu - Ala - Ile - Phe
```

Formula II

Likewise, the term "N-fold inserted" in reference to the polypeptides of Formula I is used to describe a set of polypeptides wherein from 1 to N amino acids have been inserted into the sequence defined by Formula I. Preferably, the inserted amino acids are selected from the preferred groups of synonymous amino acids of the amino acids flanking the insertion; more preferably they are selected from the more preferred groups of synonymous amino acids of the amino acids flanking the insertion, and most preferably from the most preferred groups of synonymous amino acids of the amino acids flanking the insertion (see Tables I and II). Thus, for example, one subgroup of the group of 1-fold inserted peptides comprises an amino acid inserted between the N-terminal X(Pro) and the adjacent X(Met). The insertions defining the members of this subgroup are preferably selected from Pro, Ala, Gly, Thr, Met, Phe, Ile, Val, and Leu; more preferably they are selected from Ala, Pro, Met, Leu, Phe, Ile, and Val, and most preferably they are selected from Pro, Met, Ile, and Leu. Insertions can be made between any adjacent amino acids of Formula I and also at the beginning and end of the amino acid sequence. Since there are 133 possible insertion locations, and since multiple insertions can be made at the same location, a 2-fold inserted peptide of Formula I gives rise to 17,689 subgroups of peptides, and the size of each subgroup depends on the sizes of the synonymous amino acid groups of the amino acids flanking the insertions.

The term "N-fold deleted" in reference to the polypeptides of Formula I is used to describe a set of peptides having from 1 to N amino acids deleted from the sequence defined by Formula I. Thus, the set of 1-fold deleted polypeptides of Formula I consists of 132 subgroups of polypeptides each 131 amino acids in length (131-mers). Each of the subgroups in turn consists of all the 131-mers defined by the preferred, more preferred, and most preferred synonymous amino acid groups depending on the multiplicity of substitutions.

Whenever a set of polypeptides is defined in terms of both deletions with respect to the sequence of Formula I and insertions with respect to the sequence of Formula I, first the set of polypeptides defined by the insertions is formed and then for each member of that set a set of polypeptides defined by deletions is formed. The sum of the members of all the latter sets forms the set of polypeptides defined by both insertions and deletions.

Polypeptides of the invention may be part of a longer polypeptide which comprises an additional peptide linked to the N-terminus of the polypeptide of the invention. The additional peptide may be a signal sequence for directing secretion of the protein from the host organism, e.g. MFalphal signal sequence in yeast (Kurjan et al., Cell, Vol. 30, pgs. 933-943 (1982)). Or, the additional peptide may be used to facilitate the purification of the desired polypeptide, e.g. Sassenfeld et al., "A Polypeptide Fusion Designed for the Purification of Recombinant Proteins", Biotechnology, pgs. 76-81 (January 1984). In the latter case, the additional peptide contains a selective cleavage site adjacent to the N-terminus of the desired polypeptide for the later splitting of the superfluous (with respect to biological activity) amino acid sequence by either enzymatic or chemical means.

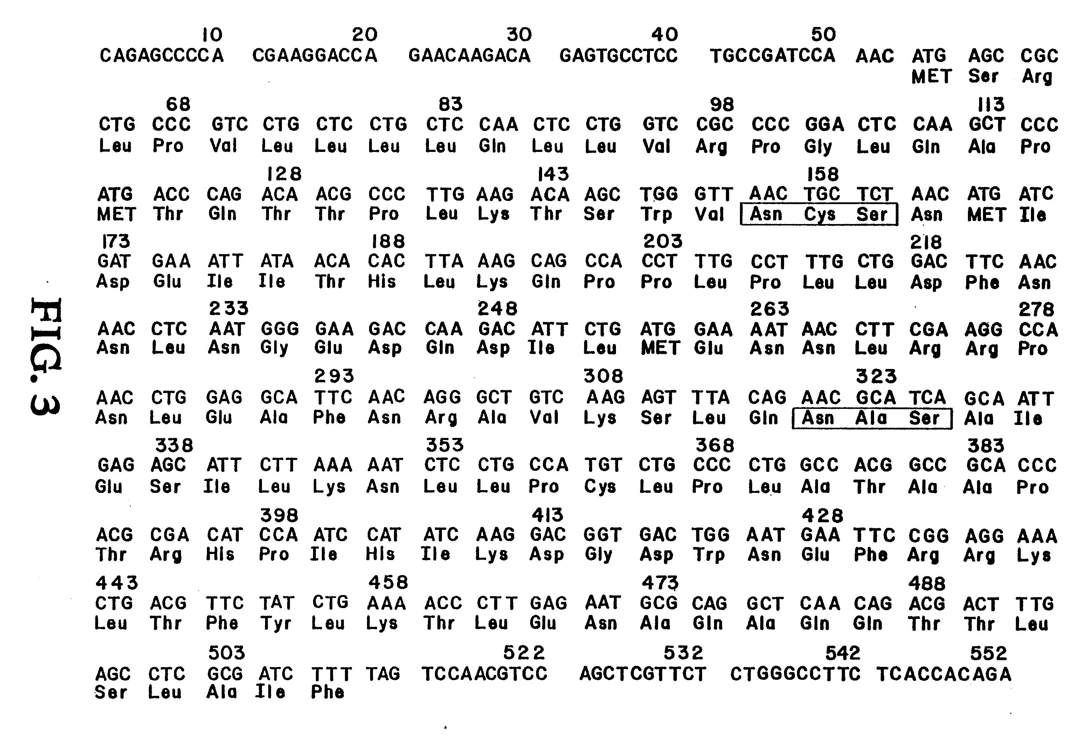

The above preferred embodiment of the invention further includes nucleotide sequences capable of encoding the polypeptides of Formula I for the preferred, more preferred, and most preferred groups of synonymous amino acids. In particular the invention includes nucleic acids having the sequence of the cDNA insert of clone pcD-huIL3-22-1 illustrated in Figure 2. pcD-huIL3-22-1 has been deposited with the American Type Culture Collection, Rockville, Maryland, under accession number 67318.

Throughout, standard abbreviations are used to designate amino acids, nucleotides, restriction endonucleases, and the like, e.g. Cohn, "Nomenclature and Symbolism of α-Amino Acids, "Methods in Enzymology, Vol. 106, pgs. 3-17 (1984); Wood et al. Biochemistry: A Problems Approach, 2nd ed. (Benjamin, Menlo Park, 1981); and Roberts, "Directory of Restriction Endonucleases", Methods in Enzymology, Vol. 68, pgs. 27-40 (1979).

The present invention is addressed to problems associated with the application of immunoregulatory agents to treat medical and/or veterinary disorders. In particular, it provides compounds capable of stimulating the regeneration of blood cells and which may be useful in cancer therapy, the treatment of certain blood diseases, and the treatment of persistent infections.

For the better understanding of the invention, preferred embodiments thereof will now be described with reference to the accompanying drawings, wherein:

Figure 1 illustrates restriction sites and major coding regions of plasmid pcDV1 used in constructing the pcD expression and cloning vector;

Figure 2 illustrates restriction sites and major coding regions of plasmid pL1 used in constructing the pcD expression and cloning vector;

Figure 3 illustrates the nucleotide sequence and predicted amino acid sequence for a human IL-3 cDNA of the invention; and

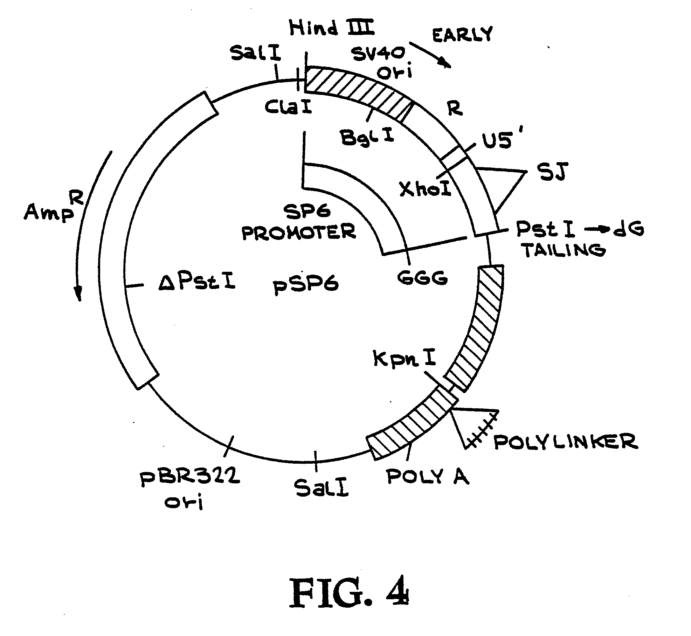

Figure 4 illustrates the modified pcD vector, pSP6, used for generating mRNA for injection into oocytes.

The present invention includes glycosylated or unglycosylated polypeptides which exhibit human IL-3 activity, and which are derivable from the IL-3 polypeptides disclosed herein using standard protein engineering techniques. The invention also includes nucleic acids having sequences capable of coding for the polypeptides of the invention. Finally, the invention includes methods of making the glycosylated or unglycosylated polypeptides of the invention which utilize the nucleotide sequences disclosed herein, and methods of using the polypeptides of the invention.

Techniques for making, using, and identifying the polypeptides and nucleic acids of the invention are discussed below in general terms. Afterwards several specific examples are provided wherein the general techniques are applied using specific cell types, vectors, reagents, and the like.

## I. Preparation of Human IL-3 cDNAs from Natural Sources

cDNAs of the present invention can be obtained by constructing a cDNA probe from the cDNA sequence disclosed in Figure 2. The probe is then used to identify a complementary cDNA clone in a cDNA library. Preferably, the cDNA library is constructed from a population of mitogen-induced T-cell clones or peripheral blood lymphocytes (PBLs). Construction of cDNA libraries from cells induced to grow and/or to synthesize certain cellular products is a well-known technique in molecular biology: e.g. recent reviews are given by Doherty, "Cloning and Expression of cDNA", Chapter 10 in Gottesman, Ed. Molecular Cell Genetics (John Wiley & Sons, New York, 1985); and Brandis et al., "Preparation of cDNA Libraries and the Detection of Specific Gene Sequences", in Setlow et al., Eds. Genetic Engineering, Vol. 8, pgs. 299-316 (Plenum Press, New York, 1986).

The extraction of total mRNA, its conversion into double-stranded cDNA, the cloning of the cDNA and the transformation of suitable hosts are carried out as outlined in the paragraph bridging pages 30 and 31 of Application PCT/US 86/02464, published as WO87/02990.

A preferred source of mRNA encoding the desired polypeptides is cells whose supernatants contain an activity associated with the polypeptides of the present invention. In general, suitable T-cells can be obtained from a variety of sources, such as human spleen, tonsils and peripheral blood. T-cell clones, such as those isolated from peripheral blood T-lymphocytes, may also be used (see Research Monographs in Immunology, eds. von Doehmer, H. and Haaf, V.; Section D: "Human T-Cell Clones", vol.8, pgs. 243-333; Elsevier Science Publishers, N.Y. [1985]).

Probes for the cDNA library are constructed using standard techniques, e.g. nick-translation of full-length or nearly full-length cDNA clones of the present invention, or by labeled synthetic oligonucleotides. Maniatis et al. (cited above) provide detailed instructions for the nick-translation technique, and labeled synthetic oligonucleotides of any desired base sequence can be constructed using commercially available DNA synthesizers, e.g. Applied Biosystems, Inc. (Foster City, CA) model 381A, or the like.

The cDNA library is then screened by the probe using standard techniques, e.g. Beltz et al., "Isolation of Multigene Families and Determination of Homologies by Filter Hybridization Methods," Methods in Enzymology, Vol. 100, pgs. 266-285 (1983); Callahan et al., "Detection and Cloning of Human DNA Sequences Related to the Mouse Mammary Tumor Virus Genome," Proc. Natl. Acad. Sci., Vol. 79, pgs. 5503-5507 (1982), Grunstein et al., Proc. Natl. Acad. Sci., Vol. 72, pgs. 3961-3965 (1975); or Benton et al., Science, Vol. 196, pgs. 180-183 (1977).

## II. Preparation of Human IL-3 Muteins by Protein Engineering

General techniques for mutating DNA and producing muteins are described in Application PCT/US 86/02464 (mentioned above) at page 33, line 4 from the bottom to page 3, line 11; these can also be applied in the present invention.

Muteins of the natural polypeptide may be desirable in a variety of circumstances. For example, undesirable side effects might be reduced by certain muteins, particularly if the side effect activity is associated with a different part of the polypeptide from that of the desired activity. Moreover, in some expression systems, the native polypeptide may be susceptible to degradation by proteases; then selected substitutions and/or deletions of amino acids which change the susceptible sequences can significantly enhance yields: e.g. British patent application 2173-804-A where Arg at position 275 of human tissue plasminogen activator is replaced by Gly or Glu. Muteins may also increase yields in purification procedures and/or increase shelf lives of proteins by eliminating amino acids susceptible to oxidation, acylation, alkylation, or other chemical modifications. For example, methionine readily undergoes oxidation to form a sulfoxide, which in many proteins causes loss of biological activity: e.g. Brot and Weissbach, Arch. Biochem. Biophys., Vol. 223, pg. 271 (1983). Often methionines can be replaced by more inert amino acids with little or no loss of biological activity: e.g. Australian patent application AU-A-52451/86. In bacterial expression systems, yields can sometimes be increased by eliminating or replacing conformationally inessential cysteine residues: e.g. Mark et al., U.S. Patent 4,518,584.

In sections below the notation used by Estell et al. (cited above) to identify muteins is followed and generalized. For example, "human IL-3 mutein Leu$^{13}$" (or simply "Leu$^{13}$" if the native protein is understood from the context) indicates a polypeptide whose amino acid sequence is identical to that of the native protein except for position 13 with respect to the N-terminus. At that position Leu has been substituted for Val. More than one substitution can be similarly indicated; e.g. a mutein having Leu substituted for Val at position 13 and Phe for Met at position 18 is referred to as human IL-3 mutein (Leu$^{13}$, Phe$^{18}$). Deletions are

indicated by "Δ's". For example, a mutein lacking Gln at position 4 is referred to as human IL-3 mutein $\Delta^4$. An insertion is indicated by "IN(Xaa)". For example, a mutein with a Leu inserted after Gln at position 4 is referred to as human IL-3 mutein $IN^4(Leu)$. Thus, human IL-3 mutein $(Ser^{10}, \Delta^4, IN^6(Gly))$ represents the native human IL-3 sequence which has been modified by replacing Thr by Ser at position 10, deleting Gln at position 4, and inserting Gly immediately after Thr at position 6. Insertion of multiple amino acids at the same site is indicated by $IN^i(Xaa_1\text{-}Xaa_2\text{-}Xaa_3\text{-}...)$, where $Xaa_1\text{-}Xaa_2\text{-}Xaa_3...$ is the sequence inserted after position i. N-terminal additions are indicated by superscript "0", e.g. $IN^0(Xaa)$; and a sequence of deletions of amino acids 6-10, for example, is designated either as $\Delta^{6-10}$ or as $(\Delta^6, \Delta^7, \Delta^8, \Delta^9, \Delta^{10})$.

Most preferably cassette mutagenesis is employed to generate human IL-3 muteins. As described more fully below, a synthetic human IL-3 gene is constructed with a sequence of unique restriction endonuclease sites spaced approximately uniformly along the gene. The uniqueness of the restriction sites should be retained when the gene is inserted into an appropriate vector, so that segments of the gene can be conveniently excised and replaced with synthetic oligonucleotides (i.e. "cassettes") which code for desired mutations.

Determination of the number and distribution of unique restriction sites entails the consideration of several factors including (1) preexisting restriction sites in the vector to be employed in expression, (2) whether species or genera-specific codon usage is desired, (3) the number of different non-vector-cutting restriction endonucleases available (and their multiplicities within the synthetic gene), and (4) the convenience and reliability of synthesizing and/or sequencing the segments between the unique restriction sites.

Generally, Formula I defines sets of polypeptides with conservative amino acid substitutions, insertions, and/or deletions with respect to the putative native human IL-3 sequence. "conservative" as used herein means (i) that the alterations are as conformationally neutral as possible, that is, designed to produce minimal changes in the tertiary structure of the mutant polypeptides as compared to the native human IL-3, and (ii) that the alterations are as antigenically neutral as possible, that is, designed to produce minimal changes in the antigenic determinants of the mutant polypeptides as compared to the native human IL-3. Conformational neutrality is desirable for preserving biological activity, and antigenic neutrality is desirable for avoiding the triggering of immunogenic responses in patients or animals treated with the compounds of the invention. While it is difficult to select with absolute certainty which alternatives will be conformationally and antigenically neutral, rules exist which can guide those skilled in the art to make alterations that have high probabilities of being conformationally and antigenically neutral, e.g. Anfisen (cited above), and Berzofsky, Science, Vol. 229, pgs. 932-940 (1985). Some of the more important rules are (1) substitution of hydrophobic residues are less likely to produce changes in antigenicity because they are likely to be located in the protein's interior, e.g. Berzofsky (cited above); (2) substitution of physiochemically similar, i.e. synonymous, residues are less likely to produce conformational changes because the replacement amino acid can play the same structural role as the substituted amino acid; and (3) alteration of evolutionarily conserved sequences is likely to produce deleterious conformational effects because evolutionary conservation suggests sequences may be functionally important.

In addition to such basic rules for selecting mutein sequences, assays are available to confirm the biological activity and conformation of the engineered molecules. Biological assays for the polypeptides of the invention are described more fully below. Changes in conformation can be tested by at least two well known assays: the microcomplement fixation method, e.g. Wasserman et al., J. Immunol., Vol. 87, pgs. 290-295 (1961), or Levine et al. Methods in Enzymology, Vol. 11, pgs. 928-936 (1967), used widely in evolutionary studies of the tertiary structures of proteins; and affinities to sets of conformation-specific monoclonal antibodies, e.g. Lewis et al., Biochemistry, Vol. 22, pgs. 948-954 (1983).

## III. CSF Activity Assays

To determine CSF activity, hemopoietic cells, e.g. bone marrow cells or fetal cord blood cells, are made into a single-cell suspension. The individual cells are then "immobilized" in a semi-solid (agar) or viscous (methylcellulose) medium containing nutrients and usually fetal calf serum. In the presence of an appropriate stimulating factor, individual cells will proliferate and differentiate. Since the initial cells are immobilized, colonies develop as the cells proliferate and mature. These colonies can be scored after 7-14 days. Detailed instructions for conducting CSF assays are given by Burgess, A., Growth Factors and Stem Cells, pgs. 52-55 (Academic Press, New York 1984), and Metcalf, The Hemopoietic Colony Stimulating Factors, pgs. 103-125 (Elsevier, New York, 1984). If desired, individual colonies can be extracted, placed on microscope slides, fixed and stained with Wright/Geimsa (Todd-Sanford, Clinical Diagnosis by Laboratory Methods, 15th Edition, Eds. Davidson and Henry [1974]). Morphological analysis of cell types present per single colony can then be determined.

Bone marrow cells collected from patients with nonhematologic disease are layered over Ficoll (type 400, Sigma Chemical Co., St. Louis, MO) and centrifuged (600 $\times$ g, 20 min), and the cells at the interface are removed. These cells are washed twice in Iscove's Modified Dulbecco's Medium containing 10% fetal calf serum (FCS) and resuspended in the same medium, and the adherent cells are removed by adherence to plastic Petri dishes (adherent cells are frequently GM-CSF producing cells (Metcalf, cited above)). The nonadherent cells are added at $10^5$ cells/ml to Iscove's Medium containing 20% FCS, 50 $\mu$m 2-mercaptoethanol, 0.9% methylcellulose and varied concentrations of either supernatants known to contain colony stimulating activity or test supernatants. One ml aliquots are plated in 35 mm petri dishes and cultured at 37°C in a fully humidified atmosphere of 6% $CO_2$ in air. Three days after the initiation of the culture, 1 unit of erythropoietin is added to each plate. Granulocyte macrophage colonies and erythroid bursts are scored at 10-14 days using an inverted microscope.

Cord blood cells collected in heparin are spun at 600 $\times$ g for 6 min. The white blood cells at the interface between the plasma and red blood cell peak are transferred to a tube containing 0.17 N ammonium chloride and 6% FCS. After 5 min on ice, the suspension is underlaid with 4 ml FCS and centrifuged for 6 minutes at 600 $\times$ g. The cell pellet is washed with Dulbecco's phosphate buffered saline and put through the Ficoll and plastic adherence steps as described above for bone marrow cells. The low-density nonadherent cells are collected and placed at $10^5$ cells/culture in the semi-solid culture medium as described above.

At the end of the assays, the cellular composition is determined after applying the individual colonies to glass slides and staining with Wright-Geimsa. Eosinophils are determined by staining with Luxol Fast Blue, e.g. Johnson, G. and Metcalf, D., Exp. Hematol., Vol. 8, pgs. 549-561 (1980).

IV. Purification and Pharmaceutical Compositions

General methods for purifying the polypeptides of the present invention, and for using them in laboratories or as active ingredients in pharmaceutical compositions, are disclosed in Section V of Application PCT/US 86/02464, especially from page 43, line 20 to page 44, line 15, and page 44, line 27 to page 45, line 27. The quantity of active polypeptide in a unit dose according to the present invention can be from 1 $\mu$g to 100 mg, according to the potency and application.

Moreover, populations of hematopoietic cells of one person may be maintained and/or expanded or caused to differentiate ex vivo for eventual reintroduction into the same or another person for a beneficial effect. The compositions can selectively stimulate various components of the immune system, either alone or with other agents well known to those skilled in the art. In particular, the compositions may include other immune-reactive agents, such as lymphokines (e.g. IL-1, IL-2, IL-4, GM-CSF, or the like).

V. Expression Systems

Any appropriate expression system can be chosen; its nature is not critical to the present invention, given that it is appropriate. Suitable expression systems are discussed in Section VI, Expression Systems, on pages 45-50 of Application PCT/US 86-02464.

EXAMPLES

The following examples serve to illustrate the present invention. Selection of cDNA libraries, vectors and hosts as well as the concentration of reagents, temperatures, and the values of other variables are only to exemplify application of the present invention and are not to be considered limitations thereof.

Example I. Preparation of Human IL-3 cDNA by Screening a pcD cDNA Library with a Synthetic Probe and Expression in COS 7 Monkey Cells and Xenopus Oocytes.

A pcD cDNA library was constructed from mRNA isolated from a human T-cell clone (6D11) induced to synthesize mRNA by exposure to concanvalin A (conA) (2-10 microgram/ml for 4-12 hours) using the techniques disclosed by Okayama and Berg, Mol. Cell. Biol., Vol. 2, pgs. 161-170 (1982) and Vol. 3, pgs.

280-289 (1983). Other induction compounds, such as phytohemagglutinin (PHA), poke weed mitogen, Calcium ionophore, anti-T3 antigen, or the like, may be more effective for some cell types and under some conditions. pcD precursor plasmids, pcDV1 and pL1, are commercially available from Pharmacia, Inc. (Piscataway, NJ).

Construction of the Okayama and Berg pcD cDNA library is briefly described in Application PC/US 86/02464 in the paragraph bridging pages 31 and 32.

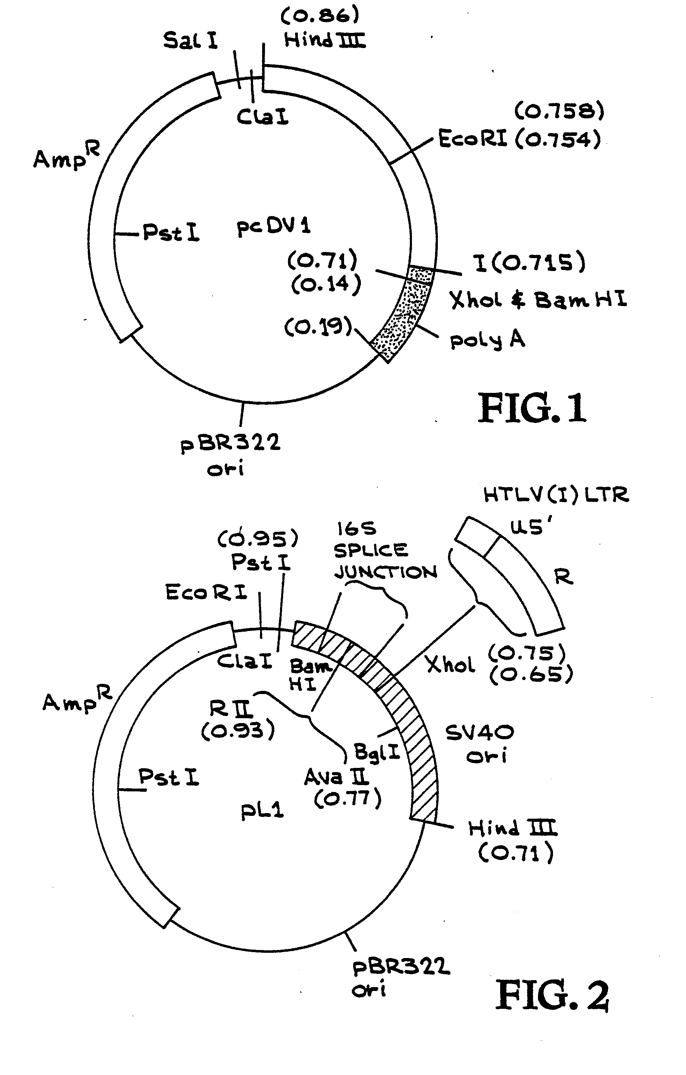

Once the cDNA library in the Okayama/berg plasmid vectors, illustrated diagrammatically in Figure 1, has been completed, the cDNA clones are collected, and random pools checked for the presence of the desired cDNAs by standard procedures, e.g. hybrid selection, detection of antigenic determinants on expressed products, and/or functional assays.

A. Isolation of mRNA

Total cellular RNA was isolated from 6D11 cells using the guanidine isothiocyanate procedure of Chirgwin et al., Biochemistry, Vol. 18, pgs. 5294-5299 (1979). The procedure is also disclosed by Maniatis et al. (cited above). Washed and pelleted cona-stimulated 6D11 cells were suspended in guanidine isothiocyanate lysis solution (50 g guanidinium thiocyanate with 0.5 g of sodium N-lauroyl sarcosine (final concentration 0.5%), 2.5 mL of 1 M sodium citrate, pH 7.0 (25 mM), 0.7 ML of 2-mercaptoethanol (0.1 M), and 0.33 mL Sigma 305 Antifoam A (0.1%); deionized water was added until the volume equaled 100 mL at room temperature, and the final volume was filtered and adjusted to pH 7 with 1 N NaOH). Twenty ml of lysis solution was used for $1.5 \times 10^8$ cells.

The resulting pellets were them processed according to the procedure of Application PCT/US 86/02464, Section B, page 55, line 22 to page 56, line 24.

B. Construction of the pcD Library

The procedure of Okayama & Berg (Mol. & Cell. Biol. Vol. 2, pgs. 161-170 [1982]) was used with only minor modifications. The pcDV1 and pL1 plasmids are described by Okayama & Berg (Mol. & Cell. Biol. 3:380-389 [1983]) and are available from Pharmacia (Piscataway, N.J.). Specifically, a modified pcDV1 plasmid was used which contained an Nsil site at the previous location of the Kpnl site, and a modified pL1 was used which contained an insertion at the Xhol site consisting of a portion of the long terminal repeat (LTR) of a HTLV(I) retro virus. The presence of the LTR segment has been found to generally enhance expression in mammalian cell hosts by a factor of 20-50. The modified constructions are illustrated in Figures 1 and 2.

Retroviral LTRs have been shown to enhance expression in a number of systems; see e.g. Chen et al., Proc. Natl. Acad. Sci., Vol. 82, pgs. 7285-7288 (1985); Gorman et al., Proc. Natl. Acad. Sci., Vol. 79, pgs. 6777-6781 (1982); Temin, Cell, Vol. 27, pgs. 1-3 (1981); and Luciw et al., Cell, Vol. 33, pgs. 705-716 (1983). The portion of the HTLV(I) LTR inserted at the Xhol site of pL1 consists of a 267 base segment including the entire R region and part of the U5 region (designated U5' in Figure 2) extending from the R/U5 boundary to the first downstream Taql site (39 bases in all). The sequence of the HTLV(I) LTR is disclosed by Yoshida et al. in Current Topics in Microbiology and Immunology, Vol. 115, pgs. 157-175 (1985).

The HTLV(I) LTR segment is inserted into pL1 in four steps utilizing procedures disclosed in Example II. First, pL1 is digested with Bgll and Xhol, and the large fragment is isolated. The large fragment is then mixed with synthetics 1A/B and 2A/B (defined below) in a standard ligation mixture. Synthetic 1A/B and 2A/B together consist of, in sequence 5'-->3", the sequence of the small Bgll/Xhol fragment, the 26 base 5' piece of the LTR R region, and a polylinker consisting of Smal, Sacl, Nael, and Xhol sites in the stated order.

```
(BglI)
   TCGGCCTCTGAGCTATTCCAG-
CGGAGCCGGAGACTCGATAAGGTC-

AAGTAGTGAGGAGGCTTTTTTGGA-
TTCATCACTCCTCCGAAAAAACCT-

GGCCTAGGCTTTT
CCGG
```

### Synthetics 1A/B

```
      ·SV40 ori —>|   R——>
      GCAAAAAGCTCGGCTCG-
ATCCGAAAACGTTTTTCGAGCCGAGC-

                    SmaI
           R ---->| SacI
CATCTCTCCTTCACGCGCCCGGGGAG-
GTAGAGAGGAAGTGCGCGGGCCCCTC-

   NaeI  XhoI
CTCGCCGGCC
GAGCGGCCGGAGCT
```

### Synthetics 2A/B

After ligation the resulting plasmid of step 1 is propagated, isolated, and digested with SmaI and SacI, and the large fragment is isolated. The large fragment is then mixed with synthetics 3A/B and 4A/B (defined below) in a standard ligation mixture.

GCCG CCC TACC TG AGG CCG CCATC-
CGG CGGG ATGG ACTC CGG CGG TAG –

CACG CCGG TTG AG TCG CG TTCT
G TG CGG CCAACTC

## Synthetics 3A/B

GCCG CCTCCCG CCTG –
AG CG CAAG ACGG CGG AGGG CGG AC–

TGG TG CCTCCTG AACTG CG TCCG C–
ACC ACGG AGG ACTTG ACG CAGG CG –

SacI
CG TCTAGG TAAG TTTAG AGCT
GCAG ATCCATTCAAATC

## Synthetics 4A/B

After ligation the resulting plasmid of step 2 is propagated, isolated, and digested with SacI and NaeI, and the large fragment is isolated. The large fragment is then mixed with synthetics 5A/B (defined below) in a standard ligation mixture.

CAGG TCG AG ACCGGG CC TTTG –
TCG AG TCCAG CTC TGG CCCGG AAAC–

TCCGG CG CTCCC TTGG AG CC TACCT–
AGG CCG CG AGGG AACCTCGG ATGG A–

NaeI
AG ACTCAGCC
TCTG AG TCGG

## Synthetics 5A/B

After ligation, the resulting plasmid of step 3 is propagated, isolated, and digested with NaeI and XhoI, and the large fragment is isolated. The large fragment is then mixed with synthetics 6A/B and 7A/B (defined below) in a standard ligation mixture to form the modified pL1 plasmid, designated pL1'.

```
NaeI
    GGCTCTCCACGCTTTGCCTGACCC-
    CCGAGAGGTGCGAAACGGACTGGG-


    TGCTTGCTCAACTCTACG
    ACGAACGAG
```

### Synthetics 6A/B

```
          TCTTTGTTTCGTTTT-
    TTGAGATGCAGAAACAAAGCAAAA-


          XhoI
    CTGTTCTGCGCCGTTACAGATC
    GACAAGACGCGGCAATGTCTAGAGCT
```

### Synthetics 7A/B

An 80 $\mu$g sample of pcDV1 DNA was digested at 30°C with 20 U of KpnI endonuclease in a reaction mixture of 450 $\mu$l containing 6 mM Tris.HCl (pH 7.5), 6 M MgCl$_2$, 6 mM NaCl, 6 mM 2-ME, and 0.1 mg of bovine serum albumin (BSA) per ml. After 16 hr the digestion was terminated with 40$\mu$l of 0.25 M EDTA (pH 8.0) and 20 $\mu$l of 10% sodium dodecyl sulfate (SDS); the DNA was recovered after extraction with water-saturated 1:1 phenol-CHCl$_3$ (hereafter referred to as phenol-CHCl$_3$) and precipitation with. ethanol. Homopolymer tails averaging 60, but not more than 80, deoxythymidylate (dT) residues per end were added to the NsiI endonuclease-generated termini with calf thymus terminal transferase as follows: The reaction mixture (38 $\mu$l) contained sodium cacodylate-30 mM Tris.HCl pH 6.8 as buffer, with 1 mM CoCl$_2$, 0.1 mM dithiothreitol, 0.25 mM dTTP, the NsiI endonuclease-digested DNA, and 68 U of the terminal deoxynucleotidyl transferase (P-L Biochemicals, Inc., Milwaukee, WI). After 30 min. at 37°C the reaction was stopped with 20 $\mu$l of 0.25 M EDTA (pH 8.0) and 10 $\mu$l of 10% SDS, and after several extractions with phenol-CHCl$_3$ the DNA was recovered by precipitation with ethanol. The DNA was then digested with 15 U of EcoRI endonuclease in 50 $\mu$l containing 10 mM Tris.HCl pH 7.4, 10 mM MgCl$_2$, 1 mM dithiothreitol, and 0.1 mg of BSA per ml for 5 hr at 37°C. The large fragment, containing the SV40 polyadenylation site and the pBR322 origin of replication and ampicillin-resistance gene, was purified by agarose (1%) gel electrophoresis and recovered from the gel by a modification of the glass powder method (Vogelstein, B. & Gillespie, D., Proc. Natl. Acad. Sci. 76: 615-619 [1979]). The dT-tailed DNA was further purified by absorption and elution from an oligo (dA)-cellulose column as follows: The DNA was dissolved in 1 ml of 10 mM Tris.HCl pH 7.3 buffer containing 1 mM EDTA and 1 M NaCl, cooled at 0°C, and applied to an oligo (dA)-cellulose column (0.6 by 2.5 cm) equilibrated with the same buffer at 0°C and eluted with water at room temperature. The eluted DNA was precipitated with ethanol and dissolved in 10 mM Tris.HCl pH 7.3 with 1 mM EDTA.

The oligo (dG) tailed linked DNA was prepared by digesting 75 $\mu$g of pL1' DNA with 20 U of PstI endonuclease in 450 $\mu$l containing 6 mM Tris.HCl pH 7.4, 6 mM MgCl$_2$, 6 mM 2-ME, 50 mM NaCl, and 0.01 mg of BSA per ml. After 16 hr at 30°C the reaction mixture was extracted with phenol-CHCl$_3$ and the DNA was precipitated with alcohol. Tails of 10 to 15 deoxyguanylate (dG) residues were then added per end with 46 U of terminal deoxynucleotidyl transferase in the same reaction mixture (38 $\mu$l) as described above, except that 0.1 mM dGTP replaced dTTP. After 20 min. at 37°C the mixture was extracted with phenol-CHCl$_3$, and the DNA was precipitated with. ethanol and then digested with 35 U of HindIII endonuclease in 50 $\mu$l containing 20 mM Tris.HCl pH 7.4, 7 mM MgCl$_2$, 60 mM NaCl, and 0.1 mg of BSA at 37°C for 4 hr. The small oligo (dG)-tailed linker DNA was purified by agarose gel (1.8%) electrophoresis and recovered as described above.

Library Construction:

This proceeded according to Section C2), cDNA Library Preparation, at page 48, line 9 from the bottom to page 61, line 10 of Application PCT/US 86/02464. Then, samples of the culture were frozen down in DMSO for storage. Separately, before addition of DMSO, twenty 15 cm culture plates were inoculated with about 3000 clones each.

Radioactively labeled probes for the above colonies were constructed as follows: (1) using the human IL-3 sequence data disclosed by Yang et al., Cell, Vol. 47, pgs. 3-10 (1986), a synthetic oligonucleotide was synthesized comprising the first 55 nucleotides of the coding strand of the reported human IL-3 sequence (starting with ATG); (2) using the same sequence data an overlapping antisense-strand oligonucleotide was synthesized comprising bases 45-100 of the reported sequence; (3) the two synthetic strands were annealed and used as primers for DNA polymerase I Klenow fragment in the presence of ATP, GTP, TTP, and radioactively labeled CTP; (4) finally the reaction was "cold chased" with unlabeled precursors to ensure maximal strand extension. ("Cold chased" means that the radioactive precursor CTP is replaced by its non-radioactive form.)

The 20 plates prepared above were screened for clones using the synthetic probes by a standard colony hybridization protocol, e.g. Maniatis et al. (cited above). Two positive colonies were detected. One of the clones, designated pcD-huIL3-22-1, was selected, amplified, and recloned into the plasmid M13 for sequencing by the dideoxy chain termination procedure, e.g. Sanger et al., Proc. Natl. Acad. Sci., Vol. 74, pgs. 5363-5367 (1977). The cDNA sequence is illustrated in Figure 3 along with the predicted amino acid sequence of the largest open reading frame. Analysis of the N-terminal amino acids according to the empirical rules published by Perlman et al., J. Mol. Biol., Vol. 167, pgs. 391-409 (1983), indicates that the third proline from the start of the open reading frame is the most likely N-terminus of the mature protein. However, in spite of these rules, the adjacent Ala may be the correct N-terminus of the native protein in view of the discovery that the mouse IL-3 N-terminus is Ala. In any case, the signal sequence is not a critical portion of the active molecule, and may comprise a wide variety of sequences with no detrimental effect on secretion or activity, e.g. Kaiser et al., Science, Vol. 235, pgs. 312-317 (1987). Potential N-glycosylation sites are enclosed by boxes in Figure 3.

Surprisingly, it was discovered that the isolated human IL-3 cDNA contained a significant amino acid difference from the sequence reported by Yang et al. (cited above): the isolated CDNA encodes a proline at position 7 (with respect to the N-terminus of Formula II) instead of a serine as reported by Yang et al. (cited above). Such a change should correspond to significant conformational differences in the two polypeptides given the dissimilarity of proline and serine.

Concurrently, the same clone was separately amplified; the pcD cloning vectors were isolated using standard techniques, e.g. Maniatis et al. (cited above); and the cDNA was expressed in two separate systems: COS 7 monkey cells and Xenopus oocytes. The pcD vectors were used to transfect COS 7 cells as follows. One day prior to transfection, approximately $10^6$ COS 7 monkey cells were seeded onto individual 100 mm plates in Dulbecco's modified Eagle's medium (DME) containing 10% fetal calf serum and 2 mM glutamine. To perform the transfection, the medium was aspirated from each plate and replaced with 4 ml of DME containing 50 mM Tris.HCl pH 7.4, 400 μg/ml DEAE-Dextran and 50 μg of the plasmid DNAs to be tested. The plates were incubated for four hours at 37°C, then the DNA-containing medium was removed, and the plates were washed twice with 5 ml of serum-free DME. DME containing 150 μM Chloroquine was added back to the plates which were then incubated for an additional 3 hrs at 37°C. The plates were washed once with DME, and then DME containing 4% fetal calf serum, 2 mM glutamine, penicillin and streptomycin was added. The cells were then incubated for 72 hrs at 37°C. The growth medium was collected and evaluated for CSF activity using the fetalcord-blood assay described above. A variety of colony types were observed.

Supernatants of Xenopus oocytes injected with RNA transcribed from the cDNA insert of pcD-huIL3-22-1 were assayed for biological activity. The protocol followed was essentially the same as disclosed by Melton et al., Nucleic Acids Research, Vol. 12, pgs. 7035-7056 (1984), and by Krieg et al., Nucleic Acids Research, Vol. 12, pgs. 7057-7070 (1984). First, the cDNA insert of pcD-huIL3-22-1 was recloned into the modified pcD vector, pSP6. pSP6 was constructed by replacing the SV40 origin of replication in pcD with the SP6 promoter, as shown in Figure 3. The modified pcD vector also contained a disabled PstI site in the ampicillin resistance gene (indicated by ΔPstI in Figure 3) and a polylinker region inserted at the XhoI site of the polyA region of pcD. The polylinker region contained EcoRI, SacI, SmaI, BamHI, XbaI, SalI, SstI, and HindIII sites. The disabled PstI site permits the SV40 ori region to be excised by digestion (prior to insertion of the polylinker) with HindIII and PstI. The appropriate SP6 promoter insert (approximately 60 bases in length) can be synthesized using standard techniques from the sequence disclosed by Melton et al. (cited above). After insertion of the SP6 promoter, the resulting plasmid was amplified, isolated, and digested with Xhol. The polylinker synthesized using standard techniques is inserted at the Xhol site. Alternatively, commercially available plasmids containing the SP6 promoter and polylinker regions can be used, e.g. pSP62-PL available from NEN Research Products (Boston, MA), or pSP18 or pSP19 available from BRL Life Technologies (Gaithersburg, MD).

Human IL-3 cDNA was excised from pcD-huIL3-22-1 by digesting with Pstl and BamHI, isolated, and ligated with Pstl/BamHI-digested pSP6 to form pSP6-huIL3. pSP6-huIL3 was amplified in E. coli MC1061, isolated, and linearized with Xbal. RNA for oocyte injection was made by transcribing the linearized pSP6-huIL3 with SP6 RNA polymerase (available from BRL Life Technologies), following the protocol of Kreig et al. (cited above) and Melton et al. (cited above). The SP6 RNA polymerase was treated with DNAse (e.g., RNAsin and RNAse-free DNAse at 1 unit/microliter and 20 micrograms/ml, respectively) and injected into the cytoplasm of the Xenopus oocytes, and the supernatant of the Xenopus cultures were assayed for CSF activity. A variety, of colony types were observed.

Example II. Construction of a Synthetic Human IL-3 Gene for Cassette Mutagenesis in pcD and Expression in COS 7 Monkey Cells

Techniques for constructing and expressing the synthetic gene of this example are standard in the art of molecular biology, e.g. especially Sproat and Gait, Nucleic Acids Research, Vol. 13, pgs. 2959-2977 (1985), and Ferretti et al., Proc. Natl. Acad. Sci., Vol. 83, pgs. 599-603 (1986); and also Mullenbach et al., J. Biol. Chem., Vol. 261, pgs. 719-722 (1986); Wells et al., Gene, Vol. 34, pgs. 315-323 (1985); and Estell et al., Science, Vol. 233, pgs. 659-663 (1986). Briefly, the synthetic human IL-3 gene is assembled from a plurality of chemically synthesized double-stranded DNA fragments. Base sequences of the synthetic gene are selected so that the assembled synthetic gene contains a series of unique restriction sites with respect to the vector carrying the gene.

The series of unique restriction sites defines a series of segments which can be readily excised and replaced with segments having altered base sequences. The synthetic fragments are inserted either directly or after ligation with other fragments into a suitable vector, such as a pcD plasmid, or the like. The above-mentioned segments correspond to the "cassettes" of Wells et al. (cited above). The synthetic fragments are synthesized using standard techniques, e.g. Gait, Oligonucleotide Synthesis: A Practical Approach (IRL Press, Oxford, UK, 1984). Preferably an automated synthesizer is employed, such as an Applied Biosystems, Inc. (Foster City, CA) model 380A. pcD plasmids and other suitable vectors are commercially available, e.g. Pharmacia. For pcD, cloning can be carried out in E. coli MC1061 or HB101, and expression can be carried out in COS monkey cells or Chinese hamster ovary cells.

In this example a synthetic human IL-3 gene is constructed for insertion into a pcD plasmid. Briefly, the pcD vector is constructed by first ligating four fragments together with T4 DNA ligase: the large HindIII/BamHI fragment from pcDV1 containing the pBR322 ori and Ampr, the HindIII/Pstl fragment from pL1 containing the SV40 early region promoter and late region introns, and synthetics 11A/B and 12A/B, described below. Next, in a series of three additional amplification, purification, and insertion steps the rest of the synthetics 13A/B through 18A/B are added until a complete synthetic IL-3 gene is present in the pcD vector.

Restriction endonuclease digestions and ligase reactions are performed using standard protocols, e.g. Maniatis et al., Molecular Cloning: A Laboratory Manual (Cold Spring Harbor Laboratory, New York, 1982).

The alkaline method (Maniatis et al., cited above) is used for small-scale plasmid preparations. For large-scale preparations a modification of the alkaline method is used in which an equal volume of isopropanol is used to precipitate nucleic acids from the cleared lysate. Precipitation with cold 2.5 M ammonium acetate is used to remove RNA prior to cesium chloride equilibrium density centrifugation and detection with ethidium bromide.

Complementary strands of synthetic DNAs to be cloned (about 400ng each) are mixed and phosphorylated with polynucleotide kinase in a reaction volume of 50 μl. This DNA is ligated with 1 μg of vector DNA digested with appropriate restriction enzymes, and ligations are in a volume of 50 μl at room temperature for 4 to 12 hours. Conditions for phosphorylation, restriction enzyme digestions, polymerase reactions, ligations, and bacterial transfections have been described (Maniatis et al., cited above).

In step 1, pcDV1 is amplified, isolated, and digested with HindIII and BamHI. The fragment containing the pBR322 ori and Amp$^r$ regions is isolated by gel electrophoresis using standard techniques, e.g. Maniatis et al. (cited above). pL1 is amplified, isolated and digested with HindIII and Pstl. The fragment containing the SV40 early region promoter is isolated by gel electrophoresis. The two isolated pcDV1 and pL1 fragments are then mixed with synthetics 11A/B and 12A/B (illustrated below) in a standard T4 DNA ligase solution. Synthetics 11A/B and 12A/B anneal to form an insert to the newly created pcD vector, which is amplified in E. coli and then isolated.

In step 2, the isolated pcD vector of step 1 is digested with SpeI and SauI. The large fragment is separated and mixed with synthetics 13A/B and 14A/B in a standard T4 DNA ligase solution to form the pcD vector of step 2, which is amplified in E. coli MC1061 and isolated.

In step 3, the isolated pcD vector of step 2 is digested with MluI and SauI. The large fragment is separated and mixed with synthetics 15A/B and 16A/B in a standard T4 DNA ligase solution to form the pcD vector of step 3, which is amplified in E. coli MC1061 and isolated.

In step 4, the isolated pcD vector of step 3 is digested with EcoRI and SauI. The large fragment is separated and mixed with synthetics 17A/B and 18A/B in a standard T4 DNA ligase solution to form the complete synthetic IL-3 gene in pcD. The pcD vector of step 4 is amplified in E. coli MC1061, isolated, and transfected into COS 7 cells as described in Example I. The culture supernatants are harvested and assayed for CSF activity as described above.

The sequences of synthetics 11A/B through 18A/B are listed below. Locations and types of unique restriction sites are indicated above the sequences.

```
(PstI)BclI
    GTG ATC AAT GAG CCG CCT GCC CG TCC TG CTC-
    ACG TC AC TAG TTAC TCGG CGG ACGGG CAGG ACG AG -


CTG CTC CAAC TCC TGG TC
G ACG AGG TT
```

## Synthetics 11A/B

```
                           NarI
        CG CCC CGG ACT CCAGG CG CCCATG ACC-
    G AGG ACC AGG CGGGG CCTG AGG TCCG CGGG TAC TGG -


                        SpeI   SauI   (BamHI)
    CAG ACAACG CCC TTG AAG ACTAG TCC TTAGGG
    G TC TG TTG CGGG AACTTC TG ATCAGG AAT CCCCTAG
```

## Synthetics 12A/B

```
     SpeI
    CTAG TTGGG TTAAC TG CTC TAACATG ATCG ATCAA-
          AACCCAATTG ACG AG ATTG TAC TAG C TAC TT-


                                           XbaI
    ATTATAACACACTTAAAG CAG CCACCTTTG CCTC TT-
    TAATATTG TG TG AATTTCG TCGG TGG AAACGG AG AA-


    CTAG ACTTCAACAACCTCAAT
    G ATC TG AAG TTG
```

## Synthetics 13A/B

GGGG AAG ACC AAG AC ATTC TG ATGG AA-
TTGG AG TTACCCC TTC TGG TTC TG TAAG AC TACC TT-

XmaIII
AATAACC TTCG ACGG CCG AACC TGG AGG CATTC AAC-
TTATTGG AAG C TG CCGGC TTGG ACC TCCG TAAG TTG –

MluI       (SauI)
AGGGC TG TC AAG AG TTTAC AG AACG CG TCC
TCCCG AC AG TTC TC AAATG TC TTG CG CAGG AAT

Synthetics 14A/B

MluI
CG CG TC AG C AATTG AG AG C ATTC TTAAAAA TC TC-
        AG TCG TTAAC TC TCG TAAG AATTTTTAG AG –

CTG CC ATG TC TG CCC
G ACGG T

Synthetics 15A/B

SacII
        C TGG CC ACCG CGG C ACCC ACG CG ACAT-
ACAG ACGGGG ACCGG TGG CG CCG TGGG TG CG CTG TA-

CCAATCC ATATC AAGG ACGG TG AC TGG ATT-
GG TTAGG TATAG TTCC TG CC AC TG ACC TAA-

EcoRI (SauI)
G AATTCCC
CTTAAGGG AAT

Synthetics 16A/B

```
EcoRI
  TTCCGG AGG AAACTG ACG TTCTATCTG –
  AAGG CCTCCTTTG ACTG CAAG ATAG AC–


  AAAACCCTTG AG AAT
  TTTTGG
```

## Synthetics 17A/B

```
              GCGCAGG CTCAACAG ACG ACT–
  GAACTCTTACG CG TCCG AG TTG TCTGCTG A–


              NheI              (SauI)
  TTG TCGCTAG CG ATCTTTTAG CC
  AACAG CG ATCG CTAG AAAATCGG ATT
```

## Synthetics 18A/B

Example III. Construction and Expression of Human IL-3 Mutein Leu[131]

Ile at position 131 is changed to Leu to form human IL-3 mutein Leu[131]. The pcD plasmid of Example II containing the complete human IL-3 gene is digested with Saul and Nhel. The large fragment is isolated and combined with the following synthetic in a standard T4 DNA ligase solution:

```
          CTAG CG CTCTTTAG CC
            G CG AG AAATCGG ATT
```

The resulting pcD vector is amplified in E. coli MC1061, isolated, and transfected into COS 7 monkey cells in accordance with the protocols described above. After incubation for 3-4 days COS 7 culture supernatants are harvested and assayed for CSF activity.

Example IV. Construction and Expression of Human IL-3 Mutein Ile[2]

Met at position 2 is changed to Ile to form human IL-3 mutein Ile[2]. The pcD plasmid of Example II containing the complete human IL-3 gene is digested with Narl and Spel. The large fragment is isolated and combined with the following synthetic in a standard T4 DNA ligase solution:

```
          CG CCCATAACCCAG ACAACG CCC–
            GGG TATTGGG TCTG TTG CGGG –


          TTG AAG A
          AACTTC TG ATC
```

The resulting pcD vector is amplified in E. coli MC1061, isolated, and transfected into COS 7 monkey cells in accordance with the protocols described above. After incubation for 3-4 days COS 7 culture supernatants are harvested and assayed for CSF activity.

The descriptions of the foregoing embodiments of the invention have been presented for purpose of illustration and description only. They are not exhaustive and do not limit the invention to the precise forms disclosed.

Applicants have deposited pcd-huIL3-22-1 with the American Type Culture Collection, Rockville, MD, USA (ATCC), under accession number 67318.

**Claims**

1. A protein exhibiting colony stimulating factor activity comprising a glycosylated or unglycosylated 10-fold substituted polypeptide defined by the formula:

```
X(Pro) - X(Met) - X(Thr) - X(Gln) - X(Thr) - W(Thr) -
                             10
W(Pro) - W(Leu) - W(Lys) - X(Thr) - X(Ser) - X(Trp) -

X(Val) - X(Asn) -   Cys   - X(Ser) - W(Asn) - W(Met) -
            20
W(Ile) - W(Asp) - W(Glu) - W(Ile) - W(Ile) - W(Thr) -
                                                 30
W(His) - W(Leu) - W(Lys) - W(Gln) - W(Pro) - W(Pro) -

X(Leu) - X(Pro) - X(Leu) - X(Leu) - X(Asp) - X(Phe) -
                             40
X(Asn) - X(Asn) - W(Leu) - W(Asn) - W(Gly) - W(Glu) -

W(Asp) - W(Gln) - W(Asp) - W(Ile) - W(Leu) - W(Met) -
            50
W(Glu) - W(Asn) - W(Asn) - W(Leu) - W(Arg) - W(Arg) -
                                                 60
W(Pro) - W(Asn) - W(Leu) - W(Glu) - W(Ala) - W(Phe) -

W(Asn) - W(Arg) - W(Ala) - W(Val) - W(Lys) - W(Ser) -
                             70
W(Leu) - W(Gln) - W(Asn) - W(Ala) - W(Ser) - W(Ala) -

W(Ile) - W(Glu) - W(Ser) - W(Ile) - W(Leu) - X(Lys) -
            80
X(Asn) - X(Leu) - X(Leu) - X(Pro) -   Cys   - X(Leu) -
                                                 90
X(Pro) - X(Leu) - X(Ala) - X(Thr) - X(Ala) - W(Ala) -

W(Pro) - W(Thr) - W(Arg) - W(His) - W(Pro) - W(Ile) -
                             100
W(His) - W(Ile) - W(Lys) - W(Asp) - W(Gly) - W(Asp) -

W(Trp) - W(Asn) - W(Glu) - W(Phe) - W(Arg) - W(Arg) -
            110
W(Lys) - W(Leu) - X(Thr) - X(Phe) - X(Tyr) - X(Leu) -
                                                 120
X(Lys) - W(Thr) - W(Leu) - W(Glu) - W(Asn) - W(Ala) -

W(Gln) - W(Ala) - W(Gln) - W(Gln) - X(Thr) - X(Thr) -
                             130
X(Leu) - X(Ser) - X(Leu) - X(Ala) - X(Ile) - X(Phe)
```

wherein:

X(Ser) represents Ser, Cys, Thr, Gly, or Asn;
X(Arg) represents Arg, His, Gln, Lys, or Glu;
X(Leu) represents Leu, Ile, Phe, Tyr, Met, or Val;
X(Pro) represents Pro or Ala;
X(Thr) represents Thr, Pro, Ser, Ala, Gly, His, or Gln;
X(Ala) represents Ala, Gly, Thr, or Pro;
X(Val) represents Val, Met, Tyr, Phe, Ile, or Leu;
X(Gly) represents Gly, Ala, Thr, Pro, or Ser;
X(Ile) represents Ile, Met, Tyr, Phe, Val, or Leu;
X(Phe) represents Phe, Trp, Met, Tyr, Ile, Val, or Leu;
X(Tyr) represents Tyr, Trp, Met, Phe, Ile, Val, or Leu;
X(His) represents His, Glu, Lys, Gln, Thr, or Arg;
X(Gln) represents Gln, Glu, Lys, Asn, His, Thr, or Arg;
X(Asn) represents Asn, Glu, Asp, Gln, or Ser;
X(Lys) represents Lys, Glu, Gln, His, or Arg;
X(Asp) represents Asp, Glu, or Asn;
X(Glu) represents Glu, Asp, Lys, Asn, Gln, His, or Arg;
X(Met) represents Met, Phe, Ile, Val, Leu, or Tyr;
X(Trp) is Trp;
W(Ser) is Ser;
W(Arg) represents Arg, His, or Lys;
W(Leu) represents Leu, Ile, Phe, or Met;
W(Pro) represents Pro or Ala;
W(Thr) is Thr;
W(Ala) represents Ala or Pro;
W(Val) represents Val, Met, or Ile;
W(Gly) is Gly;
W(Ile) represents Ile, Met, Phe, Val, or Leu;
W(Phe) represents Phe, Met, Tyr, Ile, or Leu;
W(Tyr) represents Tyr or Phe;
W(His) represents His, Gln, or Arg;
W(Gln) represents Gln, Glu, or His;
W(Asn) represents Asn or Asp;
W(Lys) represents Lys or Arg;
W(Asp) represents Asp or Asn;
W(Glu) represents Glu or Gln;
W(Met) represents Met, Phe, Ile, Val, or Leu;
W(Trp) is Trp;

and wherein regions of said polypeptide defined by amino acids 10-32 inclusive, 47-60 inclusive, 71-86 inclusive, and 95-113 inclusive are together not more than 3-fold substituted.

2. The protein of claim 1 wherein said polypeptide is 5-fold substituted such that said regions defined by amino acids 10-32 inclusive, 47-60 inclusive, 71-86 inclusive, and 95-113 inclusive are together not more than 1-fold substituted.

3. The protein of claim 2 wherein said polypeptide is 2-fold substituted such that said regions defined by amino acids 10-32 inclusive, 47-60 inclusive, 71-86 inclusive, and 95-113 inclusive undergo no substitutions.

4. The protein of claim 1 wherein:

X(Ser) is Ser;
X(Arg) represents Arg, His, or Lys;
X(Leu) represents Leu, Ile, Phe, or Met;
X(Pro) represents Pro or Ala;
X(Thr) is Thr;
X(Ala represents Ala or Pro;
X(Val) represents Val, Met, or Ile;

23

X(Gly) is Gly;

X(Ile) represents Ile, Met, Phe, Val, or Leu;

X(Phe) represents Phe, Met, Tyr, Ile, or Leu;

X(Tyr) represents Tyr or Phe;

X(His) represents His, Gln, or Arg;

X(Gln) represents Gln, Glu, or His;

X(Asn) represents Asn or Asp;

X(Lys) represents Lys or Arg;

X(Asp) represents Asp or Asn;

X(Glu) represents Glu or Gln;

X(Met) represents Met, Phe, Ile, Val, or Leu;

W(Arg) is Arg;

W(Leu) represents Leu, Ile, or Met;

W(Pro) is Pro;

W(Ala) is Ala;

W(Val) is Val;

W(Ile) represents Ile, Met, or Leu;

W(Phe) is Phe;

W(Tyr) is Tyr;

W(His) is His;

W(Gln) is Gln;

W(Asn) is Asn;

W(Lys) is Lys;

W(Asp) is Asp;

W(Glu) is Glu; and

W(Met) represents Met, Ile, or Leu.

5. The protein of claim 4 wherein said polypeptide is 5-fold substituted such that said regions defined by amino acids 10-32 inclusive, 47-60 inclusive, 71-86 inclusive, and 95-113 inclusive are together not more than 1-fold substituted.

6. The protein of claim 5 wherein said polypeptide is 2-fold substituted such that said regions defined by amino acids 10-32 inclusive, 47-60 inclusive, 71-86 inclusive, and 95-113 inclusive undergo no substitutions.

7. The protein of claim 4 wherein:

X(Arg) is Arg;

X(Leu) represents Leu, Ile, or Met;

X(Pro) is Pro;

X(Ala) is Ala;

X(Val) is Val;

X(Ile) represents Ile, Met, or Leu;

X(Phe) is Phe;

X(Tyr) is Tyr;

X(His) is His;

X(Gln) is Gln;

X(Asn) is Asn;

X(Lys) is Lys;

X(Asp) is Asp;

X(Glu) is Glu;

X(Met) represents Met, Ile, or Leu;

W(Leu) is Leu;

W(Ile) is Ile; and

W(Met) is Met.

8. The protein of claim 7 wherein said polypeptide is 5-fold substituted such that said regions defined by amino acids 10-32 inclusive, 47-60 inclusive, 71-86 inclusive, and 95-113 inclusive are together not more than 1-fold substituted.

9. The protein of claim 8 wherein said glycosylated or unglycosylated polypeptide is 2-fold substituted such that said regions defined by amino acids 10-32 inclusive, 47-60 inclusive, 71-86 inclusive, and 95-113 inclusive undergo no substitutions.

10. The protein of claim 9 wherein said glycosylated or unglycosylated polypeptide comprises a sequence of amino acids defined by the formula:

```
Pro - Met - Thr - Gln - Thr - Thr - Pro - Leu -
      10
Lys - Thr - Ser - Trp - Val - Asn - Cys - Ser -
                  20
Asn - Met - Ile - Asp - Glu - Ile - Ile - Thr -
                              30
His - Leu - Lys - Gln - Pro - Pro - Leu - Pro -
                                          40
Leu - Leu - Asp - Phe - Asn - Asn - Leu - Asn -

Gly - Glu - Asp - Gln - Asp - Ile - Leu - Met -
      50
Glu - Asn - Asn - Leu - Arg - Arg - Pro - Asn -
                  60
Leu - Glu - Ala - Phe - Asn - Arg - Ala - Val -
                              70
Lys - Ser - Leu - Gln - Asn - Ala - Ser - Ala -
                                          80
Ile - Glu - Ser - Ile - Leu - Lys - Asn - Leu -

Leu - Pro - Cys - Leu - Pro - Leu - Ala - Thr -
      90
Ala - Ala - Pro - Thr - Arg - His - Pro - Ile -
                  100
His - Ile - Lys - Asp - Gly - Asp - Trp - Asn -
                              110
Glu - Phe - Arg - Arg - Lys - Leu - Thr - Phe -
                                          120
Tyr - Leu - Lys - Thr - Leu - Glu - Asn - Ala -

Gln - Ala - Gln - Gln - Thr - Thr - Leu - Ser -
      130
Leu - Ala - Ile - Phe.
```

11. A protein exhibiting colony stimulating factor activity comprising a glycosylated or unglycosylated 5-fold inserted, 5-fold deleted, and 5-fold substituted polypeptide having a sequence of amino acids defined by the formula given in claim 1;

and wherein regions of said polypeptide defined by amino acids 10-32 inclusive, 47-60 inclusive, 71-86 inclusive, and 95-113 inclusive are together not more than 3-fold substituted.

12. The protein of claim 11 wherein said polypeptide is 2-fold inserted, 2-fold deleted, and 2-fold substituted such that said region defined by amino acids 10-32 inclusive, 47-60 inclusive, 71-86 inclusive, and 95-113 inclusive undergo no substitutions, no deletions, and no insertions.

13. The protein of claim 12 wherein said polypeptide is defined by the formula:

```
Ala - Pro - Met - Thr - Gln - Thr - Thr - Pro -
      10
Leu - Lys - Thr - Ser - Trp - Val - Asn - Cys -
            20
Ser - Asn - Met - Ile - Asp - Glu - Ile - Ile -
                        30
Thr - His - Leu - Lys - Gln - Pro - Pro - Leu -
                                    40
Pro - Leu - Leu - Asp - Phe - Asn - Asn - Leu -

Asn - Gly - Glu - Asp - Gln - Asp - Ile - Leu -
      50
Met - Glu - Asn - Asn - Leu - Arg - Arg - Pro -
                  60
Asn - Leu - Glu - Ala - Phe - Asn - Arg - Ala -
                              70
Val - Lys - Ser - Leu - Gln - Asn - Ala - Ser -
                                          80
Ala - Ile - Glu - Ser - Ile - Leu - Lys - Asn -

Leu - Leu - Pro - Cys - Leu - Pro - Leu - Ala -
      90
Thr - Ala - Ala - Pro - Thr - Arg - His - Pro -
                  100
Ile - His - Ile - Lys - Asp - Gly - Asp - Trp -
                              110
Asn - Glu - Phe - Arg - Arg - Lys - Leu - Thr -
                                          120
Phe - Tyr - Leu - Lys - Thr - Leu - Glu - Asn -

Ala - Gln - Ala - Gln - Gln - Thr - Thr - Leu -
      130
Ser - Leu - Ala - Ile - Phe.
```

14. A nucleic acid comprising a nucleotide sequence selected from the nucleotide sequences capable of encoding 10-fold substituted polypeptides defined by the formula given in claim 1;

and wherein regions of said polypeptide defined by amino acids 10-32 inclusive, 47-60 inclusive, 71-86 inclusive, and 95-113 inclusive are together not more than 3-fold substituted.

15. The nucleic acid of claim 14 wherein said nucleotide sequence is selected from the nucleotide sequences capable of encoding a polypeptide defined by the formula given in claim 10.

16. A method for producing a polypeptide exhibiting colony stimulating factor activity, the method comprising the steps of:

(a) constructing a vector comprising a nucleotide sequence coding for said polypeptide, wherein the nucleotide sequence is capable of being expressed by a host containing the vector and wherein the nucleotide sequence is selected from the nucleotide sequences capable of encoding a 10-fold substituted polypeptide defined by formula given in claim 1;

and wherein regions of said polypeptide defined by amino acids 10-32 inclusive, 47-60 inclusive, 71-86 inclusive, and 95-113 inclusive are together not more than 3-fold substituted;

(b) incorporating the vector into the host;.

(c) maintaining the host in a culture medium under conditions suitable for expression of the nucleotide sequence into said polypeptide.

17. The method of claim 16 wherein said nucleotide sequence is selected from the nucleotide sequences capable of encoding a polypeptide defined by the formula given in claim 10.

18. The method of claim 17 further comprising the step of separating said polypeptide from said culture medium and said host.

19. A pharmaceutical composition for in vitro or in vivo stimulation of blood cell growth and development comprising a therapeutically compatible carrier and an effective amount of a protein of claim 1.

20. The pharmaceutical composition of claim 19 wherein said protein comprises a sequence of amino acids defined by the formula given in claim 10, or said formula preceded by Ala.

FIG. 1

FIG. 2

```
             10          20          30          40          50
  CAGAGCCCCA  CGAAGGACCA  GAACAAGACA  GAGTGCCTCC  TGCCGATCCA  AAC  ATG  AGC  CGC
                                                                   MET  Ser  Arg

        68                      83                      98                    113
  CTG  CCC  GTC  CTG  CTC   CTG  CTC  CAA  CTC  CTG   GTC  CGC  CCC  GGA  CTC   CAA  CTC  GCT  CCC
  Leu  Pro  Val  Leu  Leu   Leu  Leu  Gln  Leu  Leu   Val  Arg  Pro  Gly  Leu   Gln  Leu  Ala  Pro

       128                     143                     158                   173
  ATG  ACC  CAG  ACG  ACA   AAG  AGC  TGG  GTT  AAC   TGC  TCT
  MET  Thr  Gln  Thr  Thr   Lys  Ser  Trp  Val [Asn   Cys  Ser]

       173                     188                     203                   218
  GAT  GAA  ATT  ATA  ACA   CAC  TTA  AAG  CAA  GAC   CCA  CCT  TTG  CCT  CTG   AAC  ATG  GAA
  Asp  Glu  Ile  Ile  Thr   His  Leu  Lys  Gln  Asp   Pro  Pro  Leu  Pro  Leu   Asn  MET  Glu

       233                     248                     263                   278
  AAC  CTC  AAT  GGG  GAA   GAC  CAA  AAC  AAT  CTT   AAC  CTT  AGG  TTC  AAC   AGG  CCA
  Asn  Leu  Asn  Gly  Glu   Asp  Gln  Asn  Asn  Leu   Asn  Leu  Arg  Phe  Asn   Arg  Pro

       293                     308                     323
  AAC  GCA  TTC  GAG  GCA   TCA  AGG  GCT  GTC  ATG   AGT  TTA  CAG  AAC  GCA   TCA  ATT
  Asn  Ala  Phe  Glu  Ala  [Ser] Arg  Ala  Val  MET   Ser  Leu  Gln [Asn  Ala   Ser] Ile

       338                     353                     368                   383
  GAG  AGC  ATT  CTT  AAA   AAT  CTC  CTG  CCA  TGT   CTG  CCC  CTG  GCC  ACG   GCA  CCC
  Glu  Ser  Ile  Leu  Lys   Asn  Leu  Leu  Pro  Cys   Leu  Pro  Leu  Ala  Thr   Ala  Pro

       398                     413                     428
  ACG  TTC  TAT  CTG  CCA   ATC  AAG  GAC  GGT  GAC   TGG  AAT  GAA  TTC  CGG   AGG  ACG
  Thr  Phe  Tyr  Leu  Pro   Ile  Lys  Asp  Gly  Asp   Trp  Asn  Glu  Phe  Arg   Arg  Thr

       443                     458                     473                   488
  CTG  ACG  AAA  CTT  GAG   CAT  ATC  AAG  ACC  CTT   GAG  GCT  CAA  CAG  GCG   CAG  AAA
  Leu  Thr  Lys  Leu  Glu   His  Ile  Lys  Thr  Leu   Glu  Ala  Gln  Gln  Ala   Gln  Lys

       503                     522             532            542            552
  AGC  CTC  GCG  ATC  TTT   TAG  TCCAACGTCC   AGCTCGTTCT   CTGGGCCTTC   TCACCACAGA
  Ser  Leu  Ala  Ile  Phe   ***
```

FIG. 3

FIG. 4

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| A | CHEMICAL ABSTRACTS, vol. 104, no. 25, 23rd June 1986, page 507, abstract no. 223626a, Columbus, Ohio, US; & JP-A-60 207 594 (AJINOMOTO CO., INC.) 19-10-1985 * Abstract * | 1,19 | C 07 K 13/00 C 12 N 15/00 C 12 P 21/02 A 61 K 37/02 |
| D,A | CELL, vol. 47, 1986, pages 3-10, Cell Press, Cambridge, Mass., US; Y.-C. YANG et al.: "Human IL-3 (multi-CSF): identification by expression cloning of a novel hematopoietic growth factor related to murine IL-3" * Whole article, especially figure 4 * | 1,19 | |
| P,X | GENE, vol. 55, 1987, pages 115-124, Elsevier Science Publ. B.V; L. DORSSERS et al.: "Characterization of a human multilineage-colony-stimulating factor cDNA clone identified by a conserved noncoding sequence in mouse interleukin-3" * Whole article, especially figure 1 * | 1,19 | TECHNICAL FIELDS SEARCHED (Int. Cl.4) C 07 K 13/00 C 12 N 15/00 C 12 P 21/00 A 61 K 37/00 |

The present search report has been drawn up for all claims

| Place of search | Date of completion of the search | Examiner |
|---|---|---|
| THE HAGUE | 30-05-1988 | HERMANN R.R.W. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)